(19) **Europäisches Patentamt**
**European Patent Office**
**Office européen des brevets**

(11) **EP 2 666 462 B1**

(12) **EUROPEAN PATENT SPECIFICATION**

(45) Date of publication and mention
of the grant of the patent:
**11.04.2018 Bulletin 2018/15**

(51) Int Cl.:
*A61K 8/65* (2006.01)    *A61K 47/36* (2006.01)
*A61K 9/06* (2006.01)    *A61Q 19/00* (2006.01)

(21) Application number: **11856000.2**

(22) Date of filing: **17.02.2011**

(86) International application number:
**PCT/KR2011/001040**

(87) International publication number:
**WO 2012/099293 (26.07.2012 Gazette 2012/30)**

(54) **RADIATION CROSS-LINKED COLLAGEN GEL, PREPARATION METHOD THEREOF**

STRAHLUNGSVERNETZTES KOLLAGENGEL, HERSTELLUNGSVERFAHREN DAFÜR

GEL DE COLLAGÈNE RÉTICULÉ PAR RAYONNEMENT, SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2011 KR 20110005588**

(43) Date of publication of application:
**27.11.2013 Bulletin 2013/48**

(73) Proprietor: **Sewon Cellontech Co., Ltd**
**Yeongdeungpo-gu, Seoul 150-724 (KR)**

(72) Inventors:
• **YU, Ji Chul**
**Namyangju-si**
**Gyeonggi-do 472-865 (KR)**
• **YEO, Se Ken**
**Yongin-si**
**Gyeonggi-do 446-740 (KR)**
• **KIM, Tai Hyoung**
**Seoul 131-794 (KR)**
• **SHU, Dong Sam**
**Seoul 139-221 (KR)**
• **CHANG, Cheong Ho**
**Seoul 137-040 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
WO-A1-82/02716    WO-A1-96/06883
CN-A- 1 389 512    JP-A- H08 308 562
KR-A- 20090 035 620    KR-B1- 100 783 228
KR-B1- 100 848 712    US-A- 4 223 984
US-A- 4 268 131    US-A- 5 800 537

• NAOKI INOUE ET AL: "A novel collagen hydrogel cross-linked by gamma-ray irradiation in acidic pH conditions", JOURNAL OF BIOMATERIALS SCIENCE, POLYMER EDITION, vol. 17, no. 8, 1 January 2006 (2006-01-01), pages 837-858, XP55214257, ISSN: 0920-5063, DOI: 10.1163/156856206777996835
• STENZEL K H ET AL: "COLLAGEN AS A BIOMATERIAL", ANNUAL REVIEW OF BIOPHYSICS AND BIOENGINEERING, ANNUAL REVIEWS INC., PALO ALTO, CA, US, vol. 3, 1 January 1974 (1974-01-01), pages 231-253, XP000998473, ISSN: 0883-9182, DOI: 10.1146/ANNUREV.BB.03.060174.001311
• TERUO MIYATA ET AL: "EFFECTS OF ULTRAVIOLET IRRADIATION ON NATIVE AND TELOPEPTIDE-POOR COLLAGEN", BIOCHIMICA ET BIOPHYSICA ACTA, ELSEVIER, NL, vol. 229, 1 January 1971 (1971-01-01), pages 672-680, XP000106582, ISSN: 0006-3002
• RÉMI PARENTEAU-BAREIL ET AL: "Collagen-Based Biomaterials for Tissue Engineering Applications", MATERIALS, vol. 3, no. 3, 16 March 2010 (2010-03-16), pages 1863-1887, XP055146090, DOI: 10.3390/ma3031863

- TRANQUILAN-ARANILLA C ET AL: "Kappa-carrageenan-polyethylene oxide hydrogel blends prepared by gamma irradiation", RADIATION PHYSICS AND CHEMISTRY, PERGAMON, AMSTERDAM, NL, vol. 55, no. 2, 11 June 1999 (1999-06-11), pages 127-131, XP004164761, ISSN: 0969-806X, DOI: 10.1016/S0969-806X(98)00317-X
- Samuel Zalipsky ET AL: "Introduction to Chemistry and Biological Applications of Poly(ethylene glycol)" In: "Poly(ethylene glycol)", 5 August 1997 (1997-08-05), American Chemical Society, Washington, DC, XP55100928, vol. 680, pages 1-13, DOI: 10.1021/bk-1997-0680.ch001, * the whole document *
- WANG L ET AL: "Effects of nerve growth factor delivery via a collagen/nano-hydroxyapatite gel to mandibular distraction osteogenesis in a rabbit model", INTERNATIONAL JOURNAL OF ORAL AND MAXILLOFACIAL SURGERY, COPENHAGEN, DK, vol. 38, no. 5, 1 May 2009 (2009-05-01), pages 557-558, XP026047458, ISSN: 0901-5027, DOI: 10.1016/J.IJOM.2009.03.562 [retrieved on 2009-04-21]
- MASAHIKO BESSHO ET AL.: 'Radiation-induced cross-linking of gelatin by using y-rays: insoluble gelatin hydrogel formation' BULL. CHEM. SOC. JPN. vol. 80, no. 5, 2007, pages 979 - 985, XP008169767
- WILLIAM T. BRINKMAN ET AL.: 'Photo-cross-linking of Type I collagen gels in the presence of smooth muscle cells: mechanical properties, cell viability, and function' BIOMACROMOLECULES vol. 4, 2003, pages 890 - 895, XP055118877

Remarks:

The file contains technical information submitted after the application was filed and not included in this specification

**Description**

Technical Field

The present disclosure relates to a radiation-crosslinked collagen gel and the preparation method and use thereof, and more particularly to a preparation method which can prepare a formulated collagen gel using a physical crosslinking method in place of a chemical crosslinking method. In the present disclosure, collagen is formulated with a biocompatible material and crosslinked to provide a collagen hydrogel which can be used for wound dressings, graft materials and cell culture. Accordingly, the present disclosure provides an industrially convenient and safe method for preparing a collagen gel which has significantly improved quality and confidence, and thus presents a good image to consumers.

Background Art

As is well known, collagen is a structural protein which forms soft tissues such as dermis, tendon/ligament, blood vessels and the like, and hard tissues such as bones, and accounts for about 1/3 of total protein in mammals.
20 or more types of collagen are known, and about 90% thereof is type I collagen which forms the skin, tendon/ligament, bones and the like.
Collagen is a triple-stranded molecule having a molecular weight of about 300,000 dalton (100,000 dalton for each strand) and is composed of -GXY- repeats, wherein G is glycine (the smallest amino acid), and X and Y are amino acids other than glycine.
Collagen is currently used in medical applications, including hemostatic agents, wound dressings, artificial blood vessels, materials for reducing wrinkles, etc. In the case of hemostatic agents, Avitene which is a collagen powder extracted from calf skin was first developed in 1974 and has been used to date.
Collagen has various advantages, including low antigenicity, high biocompatibility and bio-absorbability, induction of cell adhesion, growth and differentiation, blood coagulation, hemostatic effects, and compatibility with other polymers.
However, products made only of collagen have low physical properties (tensile strength, elasticity, degradability, etc.), and thus are somewhat difficult to use in applications that require physical properties. For this reason, collagen products have been produced by adding other biocompatible materials (synthetic polymers, bioproteins, carbohydrates and other compounds) having sufficient tensile strength or tear strength.
In addition, collagen products are formulated with chemical compounds to improve the physical properties thereof, but most of the compounds used are harmful to the human body.
Prior art documents related to collagen products include Korean Patent Registration No. 0837858 (Application No. 2006-0129466; entitled "Method for preparing water-soluble oligopeptides from porcine skin collagen by irradiation with radiation").
Specifically, Korean Patent Registration No. 0837858 discloses a method for preparing water-soluble oligopeptides from porcine skin collagen by irradiation with radiation, the method comprising the steps of: irradiating porcine skin collagen with γ-rays at a dose of 50-300 kGy, and dissolving the irradiated porcine skin collagen in a 5-10 fold amount (W/V) of 0.05-0.1M NaCl solution to form a water-soluble collagen; and adding 0.5-1 wt% of papain enzyme to the water-soluble collagen, allowing the mixture to stand for 0.5-4 hours, and fractionating the enzyme-treated material into specific oligopeptides having molecular weights of 10,000-5,000 dalton, 5,000-3,000 dalton and 3,000-1,000 dalton, respectively.
The above-mentioned prior art will now be described in further detail.

Step 1: Making porcine skin collagen water-soluble

The step of making porcine skin collagen water-soluble comprises: a process of removing unnecessary materials (fats and impurities) from the porcine skin and washing the remaining porcine skin with water; a process of cutting the washed porcine skin to a suitable size and crushing the cut porcine skin with ultrasonic waves; and a process of irradiating the crushed porcine skin with radiation at a dose of 50-300 kGy and dissolving the irradiated porcine skin in a 5-10-fold amount (W/V) of 0.10M NaCl solution, preferably 0.05-0.1M NaCl solution. Herein, a suitable radiation dose for making the porcine skin collagen is 100 kGy or more. An existing method requires the use of large amounts of acidic and alkaline chemical materials, whereas the above-mentioned prior art can produce water-soluble low-molecular-weight materials at a very high yield (4 times or higher that of the existing method) without having to use acid or salt).

Step 2: Preparation of specific oligopeptides using enzyme

Hydrolyase such as papain is added to the irradiated porcine skin to dissociate the water-soluble component.

This step comprises: a process of adding 1.5-1 wt% of papain to the irradiated porcine skin and allowing the mixture to stand for 0.5-4 hours so as to reduce the molecular weight of the water-soluble component; fractionating the low-molecular-weight component according to molecular weight using a ultrafiltration process; a process of freeze-drying the fractionated peptide components, thereby preparing porcine skin collagen-derived specific oligopeptides.

However, the above-mentioned prior art also has a problem in that, because it is the technology of reducing rather than increasing the molecular weight of collagen, it is impossible to formulate the oligopeptides alone and to formulate the oligopeptides with biocompatible materials.

Thus, the above-mentioned prior art also has a problem in that, because it reduces the molecular weight of collagen, it cannot provide a hydrogel formulation which can be used for wound dressings, graft materials and cell culture.

As a result, the above-mentioned prior art does not provide a preparation method which is industrially applicable, convenient and safe.

Further documents teach mixed hydrogels including collagen.

KR 100 783 228 discloses a method for preparing a radiation-crosslinked collagen gel comprising mixing collagen with PVA and crosslinking the mixture by irradiation with gamma rays.

WO8202716 discloses mixed hydrogels including collagen, which are gelled using gamma rays.

KR10-0848712 discloses a hydrogel prepared from chitosan and pluronic F127, using uv irradiation.

TRANQUILAN-ARANILLA C ET AL: "Kappa-carrageenan-polyethylene oxide hydrogel blends prepared by gamma irradiation", RADIATION PHYSICS AND CHEMISTRY, PERGAMON, AMSTERDAM, NL, vol. 55, no. 2, 11 June 1999, pages 127-131 discloses hydrogel blends formed with mixtures of kappa-carrageenan and polyethylene oxide, gelation using gamma irradiation.

Samuel Zalipsky ET AL: "Introduction to Chemistry and Biological Applications of Poly(ethylene glycol)" In: "Poly(ethylene glycol)", 5 August 1997 (1997-08-05), American Chemical Society, Washington, DC discusses the biological applications of poly(ethylene glycol).

WO9606883 discloses an injectable PEO gel implant prepared using irradiation.

WANG L ET AL: "Effects of nerve growth factor delivery via a collagen/nano-hydroxyapatite gel to mandibular distraction osteogenesis in a rabbit model", INTERNATIONAL JOURNAL OF ORAL AND MAXILLOFACIAL SURGERY, COPENHAGEN, DK, vol. 38, no. 5, 1 May 2009, pages 557-558 discusses the delivery of a nerve growth factor via a collagen / nano-hydroxyapatite gel. No method of preparation is taught.

CN1389512 discloses a hydrogel comprising polyvinyl alcohol and hydroxyapatite.

Detailed Description of the Disclosure

Technical Problem

The present disclosure has been made in order to solve the above-described problems occurring in the prior art, and it is a first object of the present disclosure to prepare a crosslinked collagen by irradiating liquid collagen with radiation. A second object of the present disclosure is to prepare a formulated collagen gel using a physical crosslinking method in place of a chemical crosslinking method. A third object of the present disclosure is to provide the use of a crosslinked collagen hydrogel, formulated with a biocompatible material, for wound dressings, graft materials and cell culture. A fourth object of the present disclosure is to provide an industrially convenient and safe preparation method. A fifth object of the present disclosure is to provide a crosslinked collagen gel, which has significantly improved quality and confidence, and thus can present a good image to consumers, and the preparation method and use thereof.

Technical Solution

To achieve the above objects, the present disclosure provides a method for preparing a crosslinked collagen gel, the method comprising irradiating liquid collagen with radiation to prepare a crosslinked collagen, according to claim 1.

The present disclosure also provides a method of preparing a crosslinked material by mixing a biocompatible material and liquid collagen and irradiating the mixture with radiation, and a method of using the crosslinked material as a tissue repair material for wound healing, a skin graft, a bone graft or the like.

The present disclosure also provides a method of using a hydrogel of the present disclosure in a mixture with a cosmetic component, or using the hydrogel as a mask pack in a mixture with a cosmetic component, or using the hydrogel as a wound dressing.

The present disclosure also provides a method of using a radiation-crosslinked collagen gel, the method comprising: obtaining a partially crosslinked collagen using a low dose of radiation; pouring the partially crosslinked collagen together with a gelling solution into a mold to form a gel; pouring cells and a medium on the gel; culturing the cells on the gel; and using the cultured material (hydrogel) in a mixture with a cosmetic component, or using the hydrogel as a mask pack in a mixture with a cosmetic component, or using the hydrogel as a wound dressing, or applying the hydrogel to an injured skin, or using the hydrogel as a skin graft material.

The present disclosure also provides a method of using a radiation-crosslinked collagen gel, the method comprising: obtaining a partially crosslinked collagen using a low dose of radiation; mixing the partially crosslinked collagen with a gelling solution to form a gel; drying the gel to form a thin film; pouring cells and a medium onto the film; culturing the cells on the film; and grafting the cultured corneal cells into an eyeball.

The present disclosure also provides a method of using a radiation-crosslinked collagen gel, the method comprising: obtaining a partially crosslinked collagen using a low dose of radiation; mixing the partially crosslinked collagen with a gelling solution and cells; culturing the mixture; finely grinding the cultured material; placing the ground material in a syringe; and grafting the material in the syringe into the skin or bone by injection.

Advantageous Effects

As described above, the present disclosure provides a method of preparing a crosslinked collagen by irradiating liquid collagen with radiation.

According to the present disclosure, a formulated collagen gel can be prepared using a physical crosslinking method in place of a chemical crosslinking method.

Particularly, the present disclosure provides a method of using a crosslinked collagen hydrogel, formulated with a biocompatible material, for wound dressings, graft materials and cell culture.

Thus, the present disclosure provides an industrially convenient and safe preparation method.

Due to the above effects, the collagen gel according to the present disclosure has significantly improved quality and confidence, and thus can present a good image to consumers.

Brief Description of the Drawings

FIG. 1 is a schematic view showing crosslinking collagen using radiation.
FIGS. 2 to 6 are photographs of radiation-crosslinked collagen gels according to the present disclosure.
FIGS. 7 and 8 are photographs of a hydrogel comprising a partially crosslinked collagen according to the present disclosure.
FIGS. 9 to 11 show the degree of partial crosslinking of collagen as a function of radiation dose in the present disclosure.
FIG. 12 is a photograph of a radiation-crosslinked collagen hydrogel according to the present disclosure.
FIG. 13 is a photograph of a radiation-crosslinked collagen film according to the present disclosure.
FIGS. 14 to 16 are photographs of particles obtained from a radiation-crosslinked collagen hydrogel according to the present disclosure.
FIGS. 17 to 19 schematically show methods of gelling a radiation-crosslinked collagen for cell culture according to the present disclosure.

Mode for Carrying Out the Disclosure

Hereinafter, preferred embodiments for achieving the above effects of the present disclosure will be described in detail with reference to the accompanying drawings.

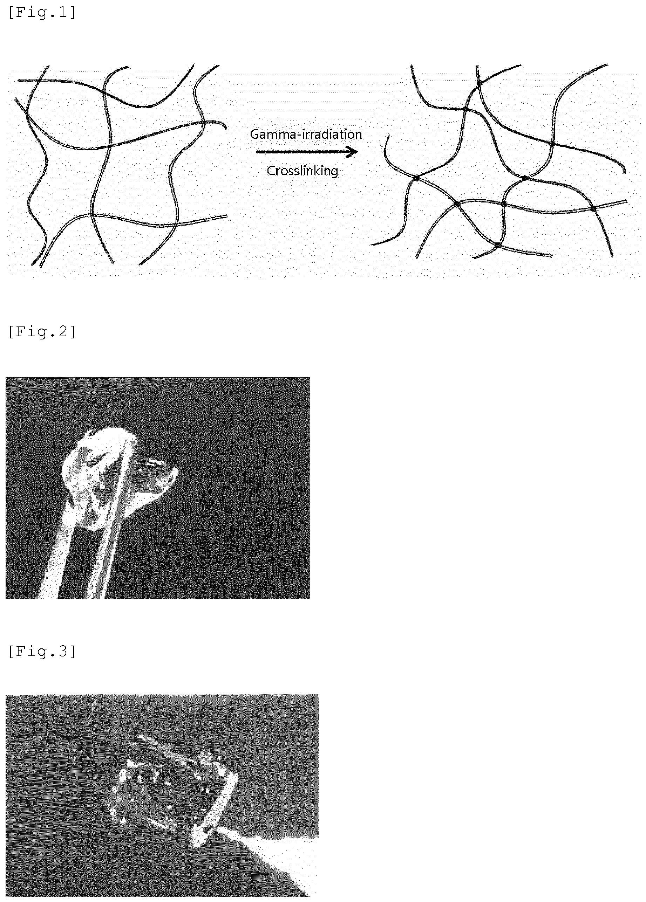

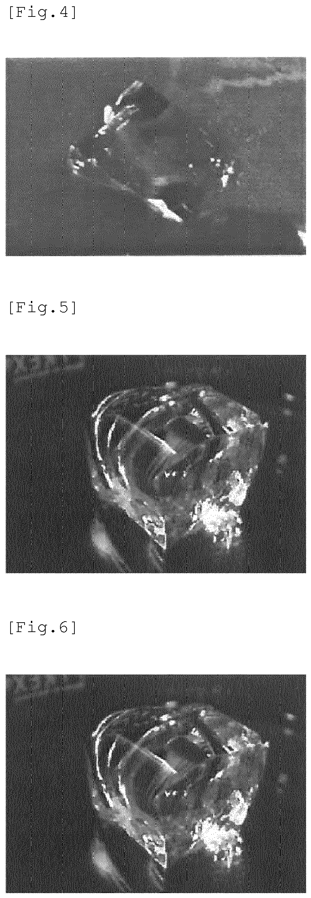

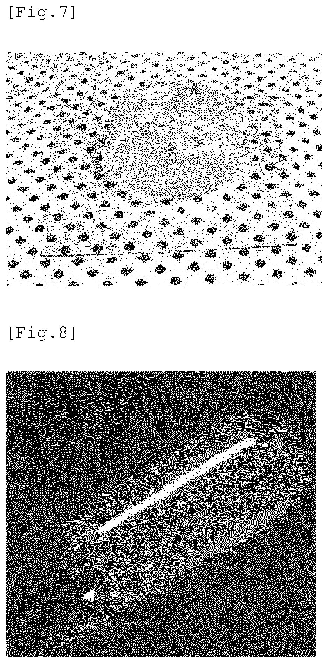

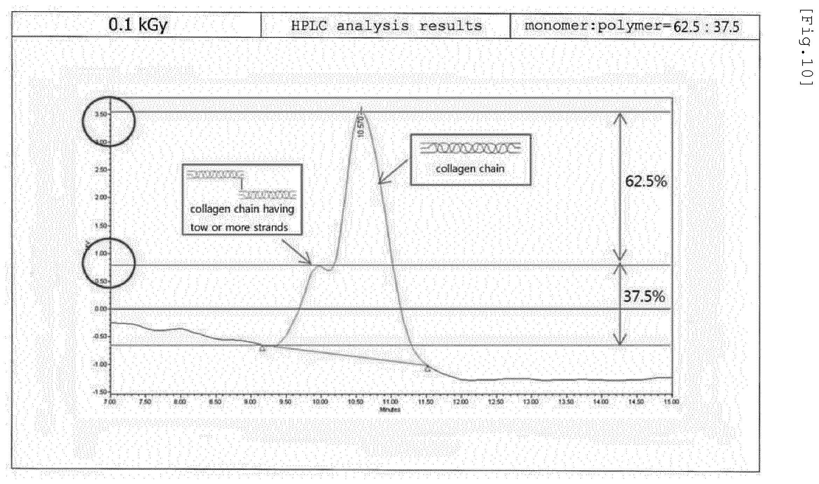

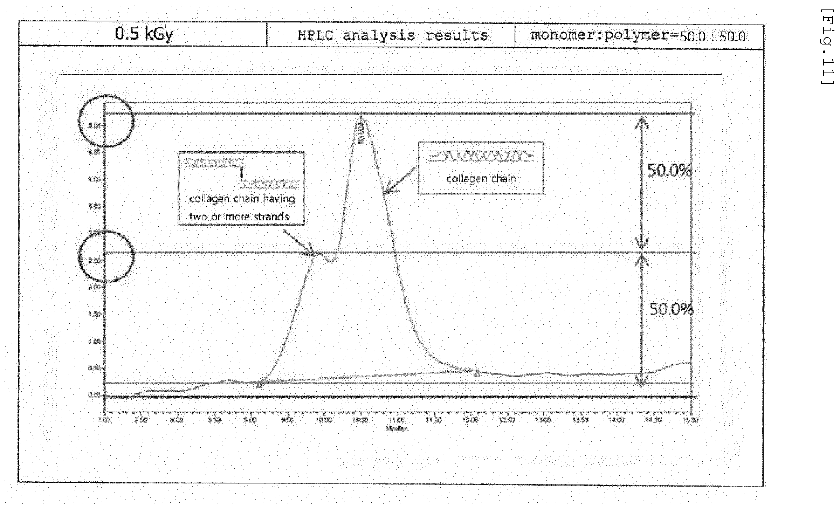

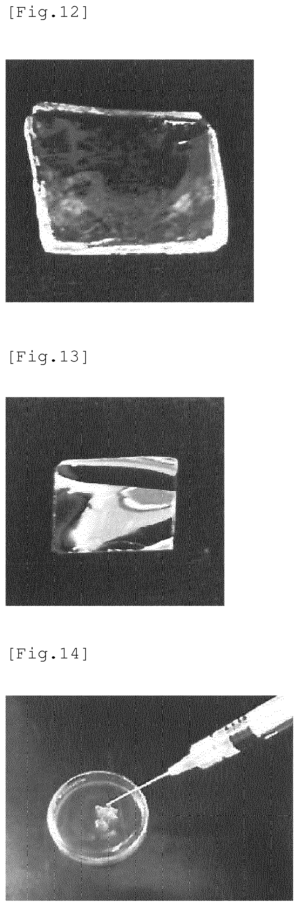

FIGS. 1 to 19 show a crosslinked collagen gel according to the present disclosure and the preparation method and use thereof.

In the following description, a detailed description of known functions and configurations incorporated herein will be omitted when it may obscure the subject matter of the present disclosure.

Also, the terms used in the following description are terms defined taking into consideration the functions obtained in accordance with the present disclosure, and may be changed in accordance with the option of a producer or a usual practice. Accordingly, definitions of these terms must be based on the overall description herein.

The technology of formulation using radiation can be explained by crosslinking of free radicals, and methods for crosslinking by radiation include gamma-ray irradiation, electron ray irradiation, X-ray irradiation and the like.

Gamma-rays are short-wavelength rays from cobalt-60 radioactive isotope and have high penetrability. Gamma-rays are used in various applications, including sterilization of products, improvement in the physical properties of polymers, and coloring of jewels.

Electron rays are electromagnetic waves having high kinetic energy, which are obtained by applying a high voltage to thermal electrons produced from tungsten electrically heated to high temperature. Electron rays are ionization energy, like gamma-rays or X-rays, and can be used in various industrial fields, and the dose thereof can be controlled depending on the intended use.

Irradiation with radiation can be performed in the absence of chemical substances without being substantially influenced by temperature, humidity or pressure. In addition, it is simply used for sterilization and is also cost-effective.

The method for crosslinking by radiation eliminates the need to remove residual toxic crosslinking agents and can cause chemical reactions even in a solid state or at low temperature, unlike chemical crosslinking methods that use formaldehyde and the like. In addition, it can easily control the physical properties of a material using a controlled dose of radiation without changing the composition of the material. Thus, methods for processing polymers using radiation have been studied and developed in various fields, including hydrogels for wound healing and burn treatment, biomaterials and the like.

According to the general theory of radiation chemistry, a solution of collagen in a water-soluble solvent is crosslinked by hydroxyl radicals (OH) produced by irradiation with radiation, and collagen can be crosslinked by radiation.

Crosslinking occurs by radical polymerization. Radical polymerization that is a chain reaction generally progresses in the order of initiation, propagation or growth, termination and movement. An initiator of radial polymerization is used to produce radicals, and a compound such as peroxide or an azo compound, which is easily controlled, is used as the initiator. In addition to the initiator compound, energy such as heat, light or radiation, is also used to produce radicals, and crosslinking can be performed without using a chemical compound. When radiation is irradiated, free radicals are produced by the supply of energy to a water molecule or a material. The free radicals influence the crosslinking of the material. The degree of crosslinking depends on the dose of radiation, and when produced free radicals are consumed, crosslinking is terminated. Thus, the degree of crosslinking of a material can be controlled by controlling the dose of radiation.

A radiation-treated material exists as a polymer solution or a hydrogel depending on the degree of crosslinking.

Collagen has the properties of a phase transition polymer. As used herein, the term "phase transition polymer" refers to a polymer whose physical properties change in response to external stimuli such as temperature, pH, electric fields and light. A polymer whose physical properties change in response to temperature is referred to as a temperature-sensitive polymer. Particularly, collagen has the properties of the temperature-sensitive polymer. Collagen exists in a liquid phase at low temperature and changes to an opaque gel phase when reaching a low critical temperature.

Specifically, at a temperature lower than the low critical solution temperature (LCST), collagen is dissolved due to a hydrogen bond with water, and the hydrogen bond is broken with increasing temperature so that the polymer units agglomerate with each other to form a gel or precipitate. When the gel is formed, the bond is a physical bond which is not a strong bond such as a covalent bond, but is attributable to an intermolecular force such as a hydrophobic bond or a polar bond. Generally, in the case of neutralized polymers, the gel is produced by hydrophobic interaction.

A collagen solution partially crosslinked by radiation also has the properties of a temperature-sensitive polymer. Particularly, when the partially crosslinked collagen solution is polymerized to form a gel, it shows a transparent/semi-transparent formulation and a relatively high elasticity.

The partially crosslinked collagen solution can be used as a tool for cell culture. When a formulation comprising general pure collagen is gelled, it shows an opaque formulation and low elasticity. In addition, the use of the partially crosslinked collagen solution can advantageously shorten the gelling time.

The partially crosslinked collagen solution can be used in the tissue engineering field, the cosmetic field and the like. Particularly, it can be used as a scaffold for cell culture in the tissue engineering field. Specifically, it can be used in a method of culturing cells using a produced collagen gel, a method of culturing cells using a produced collagen film, and a method of culturing cells using a gel formed after mixing the crosslinked collagen with cells. Particularly, the cultured cells can be used in various applications depending on the characteristics and formulations of the cells.

A collagen hydrogel obtained by irradiation with radiation is in the form of crystallized collagen. It is biocompatible and flexible and can be formulated to have required physical properties. Thus, it is used as bio-tissue in various fields, including wound dressing, cosmetic and regenerative medicine fields. A gel formed by physical crosslinking has no chemical crosslinking agent harmful to the human body, and thus is receiving attention as a medical material. Products produced by this method have advantages in that they are easily produced, are harmless to humans and environments, and can be formulated in various forms.

Thus, this polymer is a good material that can be used as a drug delivery material, a cell delivery material or an injectable extracellular matrix in the tissue engineering field.

Radiation-based technology also enables the development of medical devices. When operations to which collagen-based materials and radiation-based devices (e.g., X-ray devices or gamma knives) are applied are studied and developed, they will lead to a significant development of tissue engineering.

The radiation-crosslinked collagen gel will now be described in further detail.

As shown in FIG. 1, the present disclosure is characterized in that a crosslinked collagen is prepared by irradiating liquid collagen with radiation.

The radiation that is used in the present disclosure may be any one selected from gamma-rays, electron rays and X-rays.

The concentration of collagen that is used in the present disclosure is preferably 0.1-10% (W/V), and the dose (dose rate x time) of radiation is preferably 0.1-40 kGy.

If the collagen concentration is lower than 0.1%, the degree of crosslinking of collagen will be low so that the partially crosslinked collagen cannot be gelled, and if the collagen concentration is higher than 10%, it will difficult for current technology to mix the collagen so as to have a uniform concentration distribution. For this reason, the collagen concentration is preferably 0.1-10% (W/V).

If the dose of radiation is lower than 0.1 kGy, the degree of crosslinking of collagen will be low so that the partially crosslinked collagen cannot be gelled, and if the dose of radiation is higher than 40 kGy, collagen will be decomposed rather than being partially crosslinked. For this reason, the dose of radiation is preferably 0.1-40 kGy.

Meanwhile, a method for preparing the radiation-crosslinked collagen gel according to the present disclosure is as follows.

Specifically, the radiation-crosslinked collagen gel can be prepared by mixing 3% (W/V) of collagen with each of 3% (W/V) of synthetic polymer Pluronic F-127, 1% (W/V) of PEO (polyethylene oxide (M.W. = 100,000) and 3% (W/V) of hydroxyapatite and crosslinking the mixture by irradiation with gamma-rays.

Another method for preparing the radiation-crosslinked collagen gel according to the present disclosure is as follows.

Specifically, the radiation-crosslinked collagen gel can be prepared by mixing 3% (W/V) of collagen with each of 3% (W/V) of biopolymer hyaluronic acid (M.W. = 2,000 K) and 3% (W/V) of chondroitin sulfate and crosslinking the mixture by irradiation with gamma-rays.

Still another method for preparing the radiation-crosslinked collagen gel according to the present disclosure is as follows.

Specifically, radiation-crosslinked collagen gel can be prepared by mixing 3 % (W/V) of collagen with each of 3% (W/V) of silicone (Dow Corning, 7-9800), 6% (W/V) of glycerin and 6% (W/V) of PBS and crosslinking the mixture by irradiation with gamma-rays, in which the PBS component includes 2.8 mg of sodium phosphate and 7.6 mg of sodium chloride per mL of the final volume.

According to the above methods of the present disclosure, the radiation-crosslinked collagen gels shown in FIGS. 2 to 6 can be obtained.

The above-described configurations of the present disclosure can be modified in various forms.

It is to be understood that the present disclosure is not limited to the specific forms mentioned in the above detailed description and includes all modifications, equivalents and substitutions within the sprit and scope of the present disclosure as defined in the appended claims.

The effects of the radiation-crosslinked collagen gel according to the present disclosure and the preparation method and use thereof are as follows.

According to the present disclosure, a formulated collagen gel can be prepared using a physical crosslinking method in place of a chemical crosslinking method. Particularly, a crosslinked collagen gel formulated with a biocompatible material can be used for wound dressings, graft materials and cell culture. Thus, the present disclosure provides an industrially convenient and safe method for preparing the crosslinked collagen gel.

Example 1 (not according to the claim)

A. Analysis of the degree of crosslinking of 3-10% collagen as a function of radiation dose (5-40 kGy)

Purpose: to analyze physical properties as a function of collagen concentration and gamma-ray dose

Method

1) Each of 3%, 6% and 10% collagens is prepared in a container.
2) The collagens are irradiated with gamma-rays at doses of 5, 25 and 40 kGy, respectively.
3) The physical properties of the collagens crosslinked under the above conditions are analyzed.

A) Appearances (transparency and gelling tendency) are visually observed.
B) The shrinkage rate (percent decrease in volume) of hydrogel is examined.
C) The gel strength (maximum stress) is measured using a measurement instrument.

- Instrument: Rheo meter (CR-500DX).
- Conditions: measurement item (gel strength), penetration distance (2.5 mm), table speed (50 mm/min), and adaptor (No. 1 Φ15mm).

D) Degradability is determined as the number of days during which the gel remains in the presence of collagenase.

- Collagenase concentration: 0.1 mg/mL in PBS

E) The water content (change in water content) of crosslinked collagen is analyzed by comparing the weight after freeze drying with the weight after hydration of the crosslinked collagen.

Results

1) Appearance: transparent gel (having weak yellow at 40 kGy)
2) Shrinkage rate (percent decrease in volume) = (total weight (g) of gamma ray-irradiated sample) - weight (g) of gel mass) / total weight (g) of gamma ray-irradiated sample X 100

Table 1

| Shrinkage rate (%) | Gamma-ray dose (kGy) | | |
|---|---|---|---|
| Concentration (%) | 5 | 25 | 40 |
| 3 | 0.0 | 2.8 | 18.0 |
| 6 | 0.0 | 0.0 | 0.0 |
| 10 | 0.0 | 0.0 | 0.0 |

3) Gel strength (maximum stress)

Table 2

| Gel strength (N) | Gamma-ray dose (kGy) | | |
|---|---|---|---|
| Concentration (%) | 5 | 25 | 40 |
| 3 | 23.9 | 40.9 | 35.0 |
| 6 | 6.9 | 45.2 | 39.7 |
| 10 | 9.8 | 58.6 | 79.6 |

4) Degradability (days)

Table 3

| Degradability | Gamma-ray dose (kGy) | | |
|---|---|---|---|
| Concentration (%) | 5 | 25 | 40 |
| 3 | ** | ** | * |
| 6 | *** | *** | *** |
| 10 | *** | **** | **** |

Degradability (days) - * 1 day or less, ** 2 days or less, *** 7 days or less, and **** more than 7 days
5) Water content (change in water content) = (weight (g) of hydrated collagen gel - weight (g) of freeze-dried crosslinked collagen gel) / weight (g) of freeze-dried crosslinked collagen gel

Table 4

| Water content (change in water content, fold) | Gamma-ray dose (kGy) | | |
|---|---|---|---|
| Concentration (%) | 5 | 25 | 40 |
| 3 | 36.9 | 26.3 | 26.7 |
| 6 | 20.2 | 8.3 | 21.1 |
| 10 | 18.8 | 9.1 | 8.8 |

Example 2 (not according to the claim)

B. Analysis of the degree of crosslinking of 3-10% gelatin as a function of radiation dose (5-40 kGy)

Purpose: to analyze physical properties as a function of gelatin concentration and gamma-ray dose

Method

1) Each of 3%, 6% and 10% gelatins is prepared in a container.
2) The gelatins are irradiated with gamma-rays at doses of 5, 25 and 40 kGy, respectively.
3) The physical properties of the collagens crosslinked under the above conditions are analyzed.

A) Appearances (transparency and gelling tendency) are visually observed.
B) The shrinkage rate (percent decrease in volume) of hydrogel is examined.
C) The gel strength (maximum stress) is measured using a measurement instrument.

- Instrument: Rheo meter (CR-500DX).
- Conditions: measurement item (gel strength), penetration distance (2.5 mm), table speed (50 mm/min), and adaptor (No. 1 Φ15mm).

D) Degradability is determined as the number of days during which the gelatin remains in the presence of collagenase.

- Collagenase concentration: 0.1 mg/mL in PBS

E) The water content (change in water content) of crosslinked collagen is analyzed by comparing the weight after freeze drying with the weight after hydration of the crosslinked collagen.

Results

1) Appearance (transparency)

Table 5

| Appearance | Gamma-ray dose (kGy) | | |
|---|---|---|---|
| Concentration (%) | 5 | 25 | 40 |
| 3 | ** | * | * |
| 6 | *** | *** | *** |
| 10 | **** | **** | **** |

Appearance (transparency) - * opaque, ** semi-transparent, *** transparent, **** transparent (yellowish)
2) Shrinkage rate (percent decrease in volume) = (total weight (g) of gamma ray-irradiated sample) - weight (g) of gel mass) / total weight (g) of gamma ray-irradiated sample X 100

Table 6

| Shrinkage rate (%) | Gamma-ray dose (kGy) | | |
|---|---|---|---|
| Concentration (%) | 5 | 25 | 40 |
| 3 | 4.8 | 31.0 | 35.1 |
| 6 | 2.1 | 22.1 | 32.5 |
| 10 | 0.0 | 9.9 | 15.6 |

3) Gel strength (maximum stress)

Table 7

| Gel strength (N) | Gamma-ray dose (kGy) | | |
|---|---|---|---|
| Concentration (%) | 5 | 25 | 40 |
| 3 | 4.5 | 2.9 | 2.6 |
| 6 | 4.9 | 9.6 | 7.1 |
| 10 | 3.2 | 4.8 | 8.6 |

4) Degradability (days)

Table 8

| Degradability | Gamma-ray dose (kGy) | | |
|---|---|---|---|
| Concentration (%) | 5 | 25 | 40 |
| 3 | * | * | * |
| 6 | * | * | * |
| 10 | * | * | * |

Degradability (days) - * 1 day or less, ** 2 days or less, *** 7 days or less, and **** more than 7 days

5) Water content (change in water content) = (weight (g) of hydrated collagen gel - weight (g) of freeze-dried crosslinked collagen gel) / weight (g) of freeze-dried crosslinked collagen gel

Table 9

| Water content (change in water content, fold) | Gamma-ray dose (kGy) | | |
|---|---|---|---|
| Concentration (%) | 5 | 25 | 40 |
| 3 | 33.1 | 25.7 | 26.2 |
| 6 | 25.9 | 11.7 | 15.1 |
| 10 | 13.1 | 9.8 | 9.3 |

Example 3

C. Material prepared by irradiating a mixture of collagen and a synthetic polymer with radiation

Purpose: to examine whether a mixture of collagen and a synthetic polymer can be formulated.

Method

1) 3% (w/v) collagen is mixed with each of 3 wt% (w/v) Pluronic F-127, 1% (w/v) PEO (polyethylene oxide (M.W. = 100,000) and 3% (w/v) hydroxyapatite, and then crosslinked by gamma-rays (5 and 25 kGy).
2) The physical properties of the crosslinked mixture are analyzed.

A) Appearances (transparency and gelling tendency) are visually observed.
B) The shrinkage rate (percent decrease in volume) of the mixture is examined.
C) The gel strength (maximum stress) is measured using a measurement instrument.

- Instrument: Rheo meter (CR-500DX).
- Conditions: measurement item (gel strength), penetration distance (2.5 mm), table speed (50 mm/min), and adaptor (No. 1 Φ15mm).

D) Degradability is determined as the number of days during which the crosslinked mixture remains in the presence of collagenase.

- Collagenase concentration: 0.1 mg/mL in PBS

E) The water content (change in water content) of the crosslinked mixture is analyzed by comparing the weight after freeze drying with the weight after hydration of the crosslinked mixture.

Results

1) Appearance (transparency)

Table 10

| Appearance | Synthetic polymer mixed with collagen | | |
|---|---|---|---|
| Gamma-ray dose (kGy) | Hydroxyapatite | PEO (M.W.=100K) | Pluronic F-127 |
| 5 | ** (Hap distribution) | ** | ** |
| 25 | - | ** | ** |

Appearance (transparency) - * opaque, ** semi-transparent, *** transparent, **** transparent (yellowish)
2) Shrinkage rate (percent decrease in volume) = (total weight (g) of gamma ray-irradiated sample) - weight (g) of gel mass) / total weight (g) of gamma ray-irradiated sample X 100

Table 11

| Shrinkage rate (percent decrease in volume) | Synthetic polymer mixed with collagen | | |
|---|---|---|---|
| Gamma-ray dose (kGy) | Hydroxyapatite | PEO (M.W.=100K) | Pluronic F-127 |
| 5 | 0.0 | 0.0 | 0.0 |
| 25 | - | 2.1 | 0.0 |

3) Gel strength (maximum stress)

Table 12

| Gel strength (maximum stress) (N) | Synthetic polymer mixed with collagen | | |
|---|---|---|---|
| Gamma-ray dose (kGy) | Hydroxyapatite | PEO (M.W.=100K) | Pluronic F-127 |
| 5 | 11.3 | 40.3 | 3.9 |

(continued)

| Gel strength (maximum stress) (N) | Synthetic polymer mixed with collagen | | |
|---|---|---|---|
| Gamma-ray dose (kGy) | Hydroxyapatite | PEO (M.W.=100K) | Pluronic F-127 |
| 25 | - | 57.3 | 16.0 |

4) Degradability (days)

Table 13

| Degradability (days) | Synthetic polymer mixed with collagen | | |
|---|---|---|---|
| Gamma-ray dose (kGy) | Hydroxyapatite | PEO (M.W.=100K) | Pluronic F-127 |
| 5 | ** | * | * |
| 25 | - | * | * |

Degradability (days) - * 1 day or less, ** 2 days or less, *** 7 days or less, and **** more than 7 days

5) Water content (change in water content) = (weight (g) of hydrated collagen gel - weight (g) of freeze-dried crosslinked collagen gel) / weight (g) of freeze-dried crosslinked collagen gel

Table 14

| Water content (change in water content, fold) | Synthetic polymer mixed with collagen | | |
|---|---|---|---|
| Gamma-ray dose (kGy) | Hydroxyapatite | PEO (M.W.=100K) | Pluronic F-127 |
| 5 | 13.6 | 22.8 | 45.6 |
| 25 | - | 22.4 | 27.0 |

Example 4 (not according to the claim)

D. Material prepared by irradiating a mixture of collagen and a biopolymer with radiation

Purpose: to examine whether a mixture of collagen and a biopolymer can be formulated.

Method

1) 3% collagen is mixed with each of 3% hyaluronic acid (M.W. = 2,000 K) and 3% chondroitin sulfate and then crosslinked by gamma-rays (5 and 25 kGy).
2) The physical properties of the crosslinked mixture are analyzed.

A) Appearances (transparency and gelling tendency) are visually observed.
B) The shrinkage rate (percent decrease in volume) of the hydrogel is examined.
C) The gel strength (maximum stress) is measured using a measurement instrument.

-    Instrument: Rheo meter (CR-500DX).
-    Conditions: measurement item (gel strength), penetration distance (2.5 mm), table speed (50 mm/min), and adaptor (No. 1 Φ15mm).

D) Degradability is determined as the number of days during which the crosslinked mixture remains in the presence of collagenase.

-    Collagenase concentration: 0.1 mg/mL in PBS

E) The water content (change in water content) of the crosslinked mixture is analyzed by comparing the weight after freeze drying with the weight after hydration of the crosslinked mixture.

Results

1) Appearance (transparency)

Table 15

| Appearance | Biopolymer mixed with collagen | |
|---|---|---|
| Gamma-ray dose (kGy) | Hyaluronic acid | Chondroitin sulfate |
| 5 | ** | * |
| 25 | ** | * |

Appearance - * opaque, ** semi-transparent, *** transparent, **** transparent (yellowish)
2) Shrinkage rate (percent decrease in volume) = (total weight (g) of gamma ray-irradiated sample) - weight (g) of gel mass) / total weight (g) of gamma ray-irradiated sample X 100

Table 16

| Shrinkage rate (percent decrease in volume) | Biopolymer mixed with collagen | |
|---|---|---|
| Gamma-ray dose (kGy) | Hyaluronic acid | Chondroitin sulfate |
| 5 | 0.0 | 0.0 |
| 25 | 0.0 | 0.0 |

3) Gel strength (maximum stress)

Table 17

| Gel strength (maximum stress) (N) | Biopolymer mixed with collagen | |
|---|---|---|
| Gamma-ray dose (kGy) | Hyaluronic acid | Chondroitin sulfate |
| 5 | 2.5 | 0.9 |
| 25 | 13.5 | 20.8 |

4) Degradability (days)

Table 18

| Degradability (days) | Biopolymer mixed with collagen | |
|---|---|---|
| Gamma-ray dose (kGy) | Hyaluronic acid | Chondroitin sulfate |
| 5 | ** | ** |
| 25 | ** | * |

Degradability (days) - * 1 day or less, ** 2 days or less, *** 7 days or less, and **** more than 7 days
5) Water content (change in water content) = (weight (g) of hydrated collagen gel - weight (g) of freeze-dried crosslinked collagen gel) / weight (g) of freeze-dried crosslinked collagen gel

Table 19

| Water content (change in water content, fold) | Biopolymer mixed with collagen | |
|---|---|---|
| Gamma-ray dose (kGy) | Hyaluronic acid | Chondroitin sulfate |
| 5 | 33.3 | 39.0 |

(continued)

| Water content (change in water content, fold) | Biopolymer mixed with collagen | |
|---|---|---|
| Gamma-ray dose (kGy) | Hyaluronic acid | Chondroitin sulfate |
| 25 | 23.6 | 20.5 |

Example 5 (not according to the claim)

E. Material prepared by irradiating a mixture of collagen and a chemical compound with radiation

Purpose: to examine whether a mixture of collagen and a chemical compound can be formulated.

Method

1) 3% collagen is mixed with each of 3% 1X silicone (Dow Corning, 7-9800), 3% glycerin and 6% PBS and then crosslinked by gamma-rays (5 kGy). The PBS component includes 2.8 mg of sodium phosphate and 7.6 mg of sodium chloride per mL of the final volume.
2) The physical properties of the crosslinked mixture are analyzed.

A) Appearances (transparency and gelling tendency) are visually observed.
B) The shrinkage rate (percent decrease in volume) of the hydrogel is examined.
C) The gel strength (maximum stress) is measured using a measurement instrument.

- Instrument: Rheo meter (CR-500DX).
- Conditions: measurement item (gel strength), penetration distance (2.5 mm), table speed (50 mm/min), and adaptor (No. 1 $\Phi$15mm).

D) Degradability is determined as the number of days during which the crosslinked mixture remains in the presence of collagenase.

- Collagenase concentration: 0.1 mg/mL in PBS

E) The water content (change in water content) of the crosslinked mixture is analyzed by comparing the weight after freeze drying with the weight after hydration of the crosslinked mixture.

Results

1) Appearance (transparency)

Table 20

| Appearance | Chemical compound mixed with collagen | | |
|---|---|---|---|
| | Silicone | Glycerin | PBS |
| Gamma-ray dose (5 kGy) | * | *** | * |

Appearance (transparency) - * opaque, ** semi-transparent, *** transparent, **** transparent (yellowish)
2) Shrinkage rate (percent decrease in volume) = (total weight (g) of gamma ray-irradiated sample) - weight (g) of gel mass) / total weight (g) of gamma ray-irradiated sample X 100

Table 21

| Shrinkage (percent decrease in volume) | Chemical compound mixed with collagen | | |
|---|---|---|---|
| | Silicone | Glycerin | PBS |
| Gamma-ray dose (5 kGy) | 0.0 | 0.0 | 0.0 |

3) Gel strength (maximum stress)

Table 22

| Gel strength (maximum stress) | Chemical compound mixed with collagen | | |
|---|---|---|---|
| | Silicone | Glycerin | PBS |
| Gamma-ray dose (5 kGy) | - | 2.1 | 11.5 |

4) Degradability (days)

Table 23

| Gel strength (maximum stress) | Chemical compound mixed with collagen | | |
|---|---|---|---|
| | Silicone | Glycerin | PBS |
| Gamma-ray dose (5 kGy) | **** | ** | *** |

Degradability (days) - * 1 day or less, ** 2 days or less, *** 7 days or less, and **** more than 7 days

5) Water content (change in water content) = (weight (g) of hydrated collagen gel - weight (g) of freeze-dried crosslinked collagen gel) / weight (g) of freeze-dried crosslinked collagen gel

Table 24

| Water content (change in water content, fold) | Chemical compound mixed with collagen | | |
|---|---|---|---|
| | Silicone | Glycerin | PBS |
| Gamma-ray dose (5 kGy) | 11.4 | 55.9 | 13.9 |

Example 6 (not according to the claim)

F. Crosslinking of collagen by electron rays

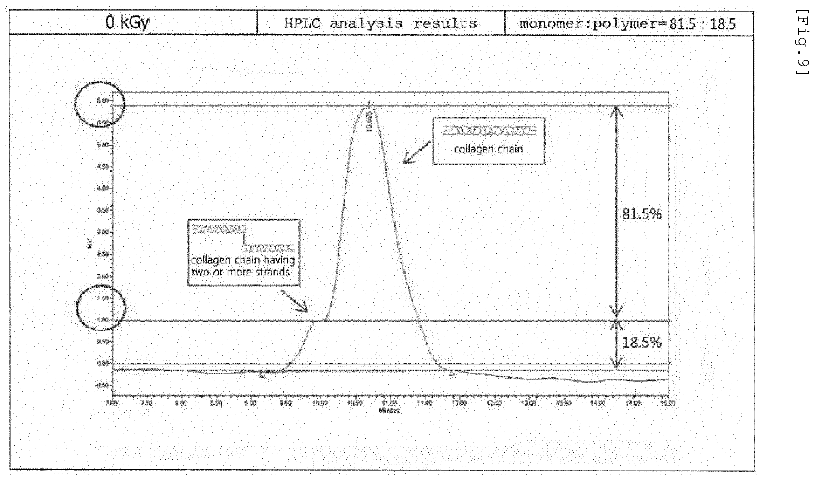

Purpose: to induce partial crosslinking of collagen by a low dose of radiation. The partially crosslinked collagen is mixed with a gelling solution, and whether the mixture is gelled is examined as a function of collagen concentration and radiation dose (FIGS. 9 to 11).

Method

1) 0.5 and 1.0% collagen solutions (pH 3.0) are prepared.
2) 1.0% collagen is irradiated with electron rays at doses of 0.1, 0.5 and 1.0 kGy.
3) 0.5% collagen is irradiated with electron rays at doses of 0.5, 1.0 and 3.0 kGy.
4) The appearances of the electron ray-irradiated samples are compared.
5) The partially crosslinked solution is mixed with a gelling solution, and whether the mixture is gelled is observed.

Gelling solution: 2.2 g $NaHCO_3$ in 100 mL of 0.05N NaOH and 200 mM HEPES.
A) The appearance of the gel is examined visually and by a Spectrophotometer (410 nm).
B) The gel strength (maximum stress) is measured with a measurement instrument.

Table 25

| Appearance of solution | Electron-ray dose (kGy) | | | |
|---|---|---|---|---|
| Concentration (%) | 0.1 | 0.5 | 1.0 | 3.0 |
| 1.0 | * | * | ** | - |
| 0.5 | - | * | *** | **** |

Appearance of solution- * transparent solution, ** transparent solution (increased viscosity), *** partial mass, and **** complete mass

1) Appearance of gel (visual observation)

Table 26

| Appearance of gel | Electron-ray dose (kGy) | | | | |
|---|---|---|---|---|---|
| Concentration (%) | 0.0 | 0.1 | 0.5 | 1.0 | 3.0 |
| 1.0 | *** | *** | *** | * | * |
| 0.5 | *** | * | ** | * | * |

Appearance of gel: not gelled, ** semi-transparent gel, and *** opaque gel.

2) Appearance of gel (spectrophotometer, 410 nm)

Table 27

| Appearance of gel | Electron-ray dose (kGy) | | |
|---|---|---|---|
| Concentration (%) | 0.0 | 0.1 | 0.5 |
| 1.0 | 2.34 | 2.22 | 1.72 |
| 0.5 | 1. 70 | - | 0.92 |

3) Gel strength (maximum stress)

| Gel strength (maximum stress, N) | Electron-ray dose (kGy) | | |
|---|---|---|---|
| Concentration (%) | 0.0 | 0.1 | 0.5 |
| 1.0 | 0.01 | 0.06 | 0.11 |
| 0.5 | 0.00 | - | 0.25 |

Example 7 (not according to the claim)

G. Examination of partial crosslinking of collagen by electron rays

Purpose: to examine whether collagen is polymerized by irradiation with electron rays.

Method

1) 1.0% collagen is irradiated with electron rays at doses of 0.1 and 0.5 kGy.
2) Each of the samples is analyzed by HPLC under the following conditions.

A) Instrument: Waters HPLC system
B) Column: Ultrahydrogel 250, 1000
C) Sample concentration: 0.1% (1 mg/mL)

D) Injection volume: 50 uL
E) Flow rate: 1 mL / min
F) Analysis time: 15 min

Example 8 (not according to the claim)

H. Hydrogel and recovery of radiation-crosslinked collagen and usability of radiation-crosslinked collagen for wound dressings, cosmetic products (mask packs) and the like.

Purpose: to examine whether radiation-crosslinked collagen can form hydrogel and can form a film when being dried. Also, to examine whether the film formulation contains water and is recovered. This experiment is a basic experiment for applying radiation-crosslinked collagen in the form of hydrogel.

Method

1) 3% and 6% collagens are prepared in dishes.
2) Each of the collagens is irradiated with gamma-rays at doses of 5 and 25 kGy.
3) Collagen hydrogels are dried at room temperature.
4) The elasticity of the film is measured, and then the film is added to distilled water to form hydrogel, and the recovery rate of the water content thereof is measured.

Results

Table 29

|  |  | Elasticity of film formulation | Water content recovery rate (%) |
|---|---|---|---|
| 3% | 5 kGy | *** | 80 |
|  | 25 kGy | *** | 30 |
| 6% | 5 kGy | ** | 100 |
|  | 25 kGy | * | 40 |

Elasticity of film formulation - * broken, ** hard, and *** bent

```
      Water  content  recovery  rate  (%)  =  (thickness  (mm)
of  water-absorbed  hydrogel  /  thickness  (mm)  of  hydrogel
before  drying
```

Example 9 (not according to the claim)

I. Use of radiation-radiated collagen as graft material

Purpose: to examine whether particles obtained from a radiation-crosslinked hydrogel formulation can be used as graft materials.

Method

1) A radiation-crosslinked hydrogel formulation is ground with a homogenizer.
2) The hydrogel is ground under the following conditions.

A) Instrument: Homogenizer (IKA T25 digital ultra-turrax)
B) Grinding conditions: rpm = 4.0-240.0, 1/min X100

3) The particles are filled into a syringe, and then injected through the needle of the syringe.

Example 10 (not according to the claim)

J. Thermal stability of radiation-crosslinked collagen

Purpose: to examine whether radiation-crosslinked collagen is thermally stable. After thermally treating the collagen, whether the collagen maintains the properties of hydrogel is also examined.

Material and test conditions

1) Material: 3% collagen (pH 3.0).
2) Crosslinking conditions: collagen solution; crosslinking by gamma-rays at doses of 5 kGy, 25 kGy and 40 kGy.
3) Heat-treatment conditions: 40, 60 and 80°C (heat-treatment time: 1 hour).

Method

1) Radiation-crosslinked collagen having a suitable size is prepared and the weight thereof is measured.
2) The prepared crosslinked collagen is placed in a container and heated in a water bath to each of the above temperatures for 1 hour.
3) After heating, the appearance and weight of the collagen gel are examined.
4) The change in weight caused by heat treatment is examined. However, whether non-crosslinked collagen is denatured is determined based on gelling in cold temperature conditions.

```
Residual rate (%) = 100 - {(initial weight -
weight after heat treatment) / initial weight * 100})
```

5) Whether the deformed collagen gel is hydrated is examined. The hydration is performed in water for 1 and 24 hours.

```
Water content recovery rate (%) = weight after
hydration / initial weight of collagen hydrogel * 100)
```

6) Whether the appearance of water-containing gel is changed at 60 °C is examined.

Results

Table 30

| 40 °C | Appearance after heat treatment | Residual rate (%) | Recovery rate (%, 1 hr) | Recovery rate (%, 24 hr) | Remarks |
|---|---|---|---|---|---|
| 0 kGy | Viscosity decreased | - | - | - | Collagen denatured |
| 5 kGy | Transparent shrunk gel | 64.5 | 128.5 | 142.3 | |
| 25 kGy | Transparent shrunk gel | 70.1 | 97.2 | 107.9 | |

(continued)

| 40 °C | Appearance after heat treatment | Residual rate (%) | Recovery rate (%, 1 hr) | Recovery rate (%, 24 hr) | Remarks |
|---|---|---|---|---|---|
| 40 kGy | Transparent shrunk gel | 73.2 | 91.3 | 104.3 | |

Table 31

| 60 °C | Appearance after heat treatment | Residual rate (%) | Recovery rate (%, 1 hr) | Recovery rate (%, 24 hr) | Remarks |
|---|---|---|---|---|---|
| 0 kGy | Viscosity decreased | - | - | - | Collagen denatured |
| 5 kGy | Flexible | - | - | - | Denatured |
| 25 kGy | Transparent shrunk gel | 33.0 | 60.4 | 85.8 | |
| 40 kGy | Transparent shrunk gel | 34.2 | 59.0 | 84.5 | |

Table 32

| 80 °C | Appearance after heat treatment | Residual rate (%) | Recovery rate (%, 1 hr) | Recovery rate (%, 24 hr) | Remarks |
|---|---|---|---|---|---|
| 0 kGy | Viscosity decreased | - | - | - | Collagen denatured |
| 5 kGy | Liquid | - | - | - | Collagen denatured |
| 25 kGy | Transparent shrunk gel | 29.0 | 64.7 | 98.6 | |
| 40 kGy | Transparent shrunk gel | 31.2 | 59.3 | 90.6 | |

Table 33

| 60 °C/containing water | Appearance after heat treatment | Residual rate (%) | Recovery rate (%, 1 hr) | Recovery rate (%, 24 hr) | Remarks |
|---|---|---|---|---|---|
| 0 kGy | Viscosity decreased | - | - | - | Collagen denatured |
| 5 kGy | Changed to Liquid | - | - | - | Collagen denatured |
| 25 kGy | Transparent shrunk gel | 55.4 | 99.5 | | |
| 40 kGy | Transparent shrunk gel | 67.2 | 99.3 | | |

The present disclosure relates to a method for crosslinking collagen by radiation and aims to provide a collagen hydrogel which is used for wound dressings and graft materials. The crosslinking is carried out at room temperature.
Specifically, according to the present disclosure, the radiation-crosslinked collagen gel is used in various manners as follows.

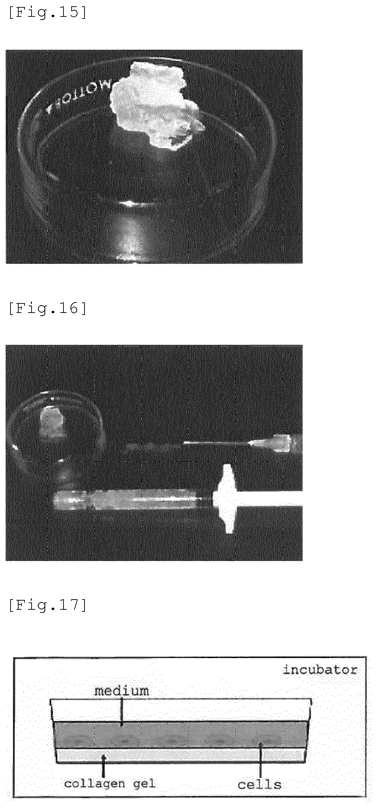

First, as shown in FIG. 17, a partially crosslinked collagen obtained using a low dose of radiation is poured into a mold together with a gelling solution to form a gel. Cells and a medium are poured on the gel, and the cells are cultured on the gel. The cultured material (hydrogel) may be used in a mixture with a cosmetic component, or mixed with a cosmetic component to provide a mask pack, or used as a wound dressing, or is applied to an injured skin, or used as a skin graft material.

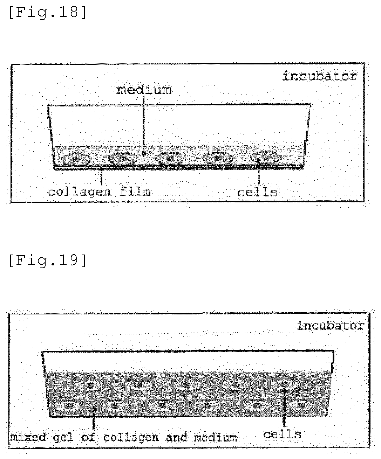

Second, as shown in FIG. 18, a partially crosslinked collagen obtained using a low dose of radiation is mixed with a gelling solution to form a gel. The gel is dried to form a thin film, after which cells and a medium are poured on the film, and the cells are cultured on the film. The cultured corneal cells may be grafted into an eyeball.

Third, as shown in FIG. 19, a partially crosslinked collagen obtained using a low dose of radiation is mixed with a gelling solution and cells, and the mixture is cultured. The cultured material may be ground finely, placed in a syringe, and grafted into the skin or bone by injection.

Herein, the gelling solution that is used in the present disclosure is preferably a mixture of sodium hydrogen carbonate ($NaHCO_3$), sodium hydroxide (NaOH) and HEPES.

Industrial Applicability

The radiation-crosslinked collagen gel according to the present disclosure, and the preparation method and use thereof show substantially reproducible results. Particularly, when the present disclosure is carried out, it can contribute to the development of industry.

**Claims**

1. A method for preparing a radiation-crosslinked collagen gel, the method comprising irradiating liquid collagen with radiation to prepare a crosslinked collagen,
   wherein the radiation is gamma-rays,
   wherein the dose (dose rate x time) of the radiation is 0.1-40 kGy/hr,
   wherein the method comprises mixing 3% (W/V) of collagen with one of 3% (W/V) of synthetic polymer Pluronic F-127® (poloxamer 407), 1% (W/V) of PEO (polyethylene oxide (M.W. = 100,000) or 3% (W/V) of hydroxyapatite, and crosslinking the mixture by irradiation with gamma-rays.

**Patentansprüche**

1. Verfahren zur Herstellung eines strahlungsvernetzten Collagen-Gels, wobei das Verfahren die Bestrahlung von flüssigem Collagen mit Strahlung zur Herstellung eines vernetzten Collagens umfasst,
   wobei es sich bei der Strahlung um Gammastrahlen handelt,
   wobei die Dosis (Dosisleistung x Zeit) der Strahlung 0,1-40 kGy/h beträgt,
   wobei das Verfahren das Mischen von 3% (w/v) Collagen mit einem aus 3% (w/v) des synthetischen Polymers Fluronic F-127® (Poloxamer 407), 1% (w/v) PEO (Polyethylenoxid (MW = 100 000)) oder 3% (w/v) Hydroxyapatit und das Vernetzen des Gemischs durch Bestrahlung mit Gammastrahlen umfasst.

**Revendications**

1. Procédé pour préparer un gel de collagène radioréticulé, le procédé comprenant l'irradiation de collagène liquide avec un rayonnement pour préparer un collagène radioréticulé,
   dans lequel le rayonnement consiste en des rayons gamma,
   dans lequel la dose (débit de dose x temps) du rayonnement est de 0,1 à 40 kGy/h,
   dans lequel le procédé comprend les étapes consistant à mélanger 3 % (p / v) de collagène avec l'un parmi 3 % (p / v) du polymère synthétique Pluronic F-127® (poloxamère 407), 1 % (p / v) de PEO (poly(oxyde d'éthylène), PM = 100 000) ou 3 % (p / v) d'hydroxyapatite, et à réticuler le mélange par irradiation avec des rayons gamma.

[Fig.1]

[Fig.2]

[Fig.3]

[Fig.4]

[Fig.5]

[Fig.6]

[Fig.7]

[Fig.8]

[Fig.9]

[Fig.10]

EP 2 666 462 B1

[Fig.11]

[Fig.12]

[Fig.13]

[Fig.14]

[Fig.15]

[Fig.16]

[Fig.17]

[Fig.18]

[Fig.19]

## REFERENCES CITED IN THE DESCRIPTION

**Patent documents cited in the description**

- KR 0837858
- KR 20060129466
- KR 100783228
- WO 8202716 A
- KR 100848712
- WO 9606883 A
- CN 1389512

**Non-patent literature cited in the description**

- Kappa-carrageenan-polyethylene oxide hydrogel blends prepared by gamma irradiation. **TRANQUI-LAN-ARANILLA C et al.** RADIATION PHYSICS AND CHEMISTRY. PERGAMON, 11 June 1999, vol. 55, 127-131
- Introduction to Chemistry and Biological Applications of Poly(ethylene glycol). **SAMUEL ZALIPSKY et al.** Poly(ethylene glycol). American Chemical Society, 05 August 1997
- **WANG L et al.** Effects of nerve growth factor delivery via a collagen/nano-hydroxyapatite gel to mandibular distraction osteogenesis in a rabbit model. *INTERNATIONAL JOURNAL OF ORAL AND MAXILLOFACIAL SURGERY,* 01 May 2009, vol. 38 (5), 557-558